# EUROPEAN PATENT APPLICATION

(11) **EP 4 455 119 A1**
(43) Date of publication of application: **30.10.2024**
(21) Application number: 22909975.9
(22) Date of filing: 20.12.2022
(51) Int. Cl.: C07C 43/23, C07C 41/03

(54) **METHOD FOR SYNTHESIZING TYLOXAPOL**

(30) Priority: 21.12.2021 CN 202111571701; 12.12.2022 CN 202211599865
(71) Applicant: Shenyang Xingqi Pharmaceutical Co., Ltd., Shenyang, Liaoning 110167 (CN)
(72) Inventor: LIU, Jidong, Shenyang, Liaoning 110163 (CN); YANG, Qiang, Shenyang, Liaoning 110163 (CN); LEI, Yu, Shenyang, Liaoning 110163 (CN); WEN, Bo, Shenyang, Liaoning 110163 (CN)
(74) Representative: Casalonga
(86) International application number: PCT/CN2022/140268
(87) International publication number: WO 2023/116663

(57) **Abstract**

The present invention provides a method for synthesizing tyloxapol of formula (I), comprising the following steps: first reacting p-tert-octylphenol of formula (III) with formaldehyde under alkaline conditions to obtain 2,5-dimethylol p-tert-octylphenol of formula (IV); then reacting 2,5-dimethylol p-tert-octylphenol with p-tert-octylphenol of formula (III) under acidic conditions to obtain the phenolic resin of formula (II); and finally, reacting the phenolic resin of formula (II) with ethylene oxide to obtain tyloxapol of formula (I).

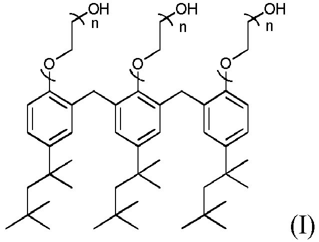

## Description

### TECHNICAL FIELD

The present disclosure belongs to the field of chemical synthesis, and particularly relates to a method for synthesizing tyloxapol.

### BACKGROUND

Tyloxapol is a high molecular polymer (as shown below), and is an alkyl aromatic polyether alcohol type non-ionic liquid surfactant, in which a hydrophobic aromatic group improves the solubility of this molecule in oil, while a large number of hydrophilic group ether alcohol increases the solubility of this molecule in water. Therefore, tyloxapol is used as an emulsifier in water/oil and oil/water emulsions.

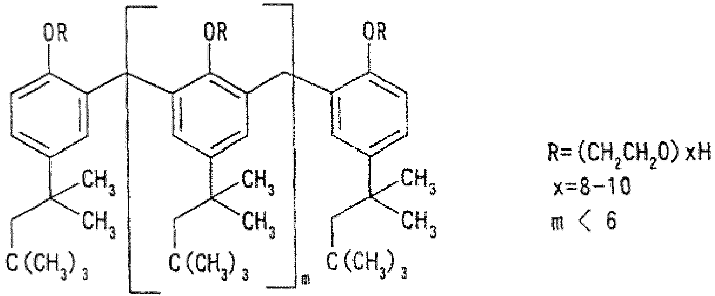

In addition to being used as an emulsifier for the preparation of emulsions, creams and other formulations, tyloxapol is also widely used in the preparation of liquid formulations, such as nose drops, eye drops, mixtures, etc. and solid formulations, such as tablets, pills, etc., due to its good solubilization and dispersion effects, which can shorten the disintegration time and improve the dissolution in tablets.

As an active ingredient in antitussive and expectorant formulations, tyloxapol is used to reduce surface tension and liquefy mucus so as to make phlegm easy to be discharged. Tyloxapol also plays the role of emulsification and penetration enhancement as an additive in the formulation. It is also reported that tyloxapol is used in eyeball cleaners (collyriums) and has good decontamination and cleaning effects. The concentration used as pharmaceutical additives should be determined according to the specific prescription, dosage form, and purpose of use. Generally, the concentration in use is 0.1 to 0.5%.

At present, the processes for synthesizing tyloxapol are mainly as follows: 1) Firstly, reacting tert-octylphenol with ethylene oxide in the presence of sodium hydroxide at high temperature and high pressure to generate polymerized phenol, and then condensing the polymerized phenol with formaldehyde in the presence of an acidic catalyst under heating conditions to prepare tyloxapol; 2) Firstly, polymerizing formaldehyde with p-tert-octylphenol to obtain a phenolic resin with a qualified molecular weight, and then reacting the qualified phenolic resin with ethylene oxide to synthesize tyloxapol. These two routes are prone to generate polymers with a polymerization degree of greater than 6 (pharmacopoeia standard m<6) during phenolic aldehyde polymerizations, which require high-temperature (above 160°C) vacuum distillation to retain fractions at specific temperatures. This is a great challenge to pilot equipments and public projects.

Therefore, there is a need in the field for tyloxapol with a degree of polymerization m<6, especially m=1, and a preparation method thereof.

### SUMMARY

In view of the shortcomings of the above synthesis methods, the present disclosure proposes a new synthesis method and prepares tyloxapol with m=1. The method of the present disclosure is simple to operate and the obtained product has high purity, thereby being beneficial to industrial production.

Specifically, the present disclosure relates to the following technical solutions:
**1.** A compound of formula (I): 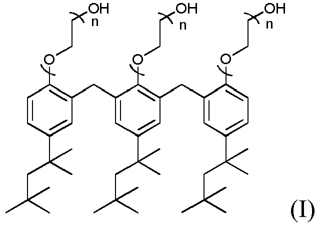 wherein n is 6, 7, 8, 9, 10, 11 or 12; alternatively, n is 8, 9 or 10; yet alternatively, n is 9.
**2.** A method for preparing the compound of formula (I) of embodiment 1, 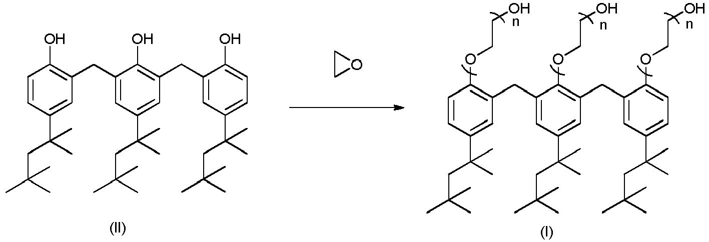 comprising the step of reacting the compound of formula (II) with ethylene oxide to obtain the compound of formula (I).
**3.** The method of embodiment 2, wherein the molar ratio of the compound of formula (II) to ethylene oxide is 1: 19 to 1:37, alternatively 1:25 to 1:32, alternatively 1:27 to 1:29, yet alternatively 1:28.
**4.** The method of embodiment 2 or 3, wherein the reaction of the compound of formula (II) with ethylene oxide is carried out in an aprotic solvent, alternatively in an aprotic non-polar solvent; alternatively, the solvent is an alkane, such as n-hexane, heptane, benzene, and toluene, still alternatively, toluene.
**5.** The method of any one of embodiments 2-4, wherein the reaction is carried out at a temperature of 60°C or more (such as 60-120°C), alternatively at a temperature of 80°C or more (such as 80-100°C), still alternatively at a temperature of 100°C.
**6.** The method of any one of embodiments 2-5, wherein the reaction is carried out at a pressure of 0.2-0.8 MPa, alternatively at a pressure of 0.3-0.8 MPa, yet alternatively at a pressure of 0.3-0.5 MPa.
**7.** The method of any one of embodiments 2-6, wherein the reaction is carried out for at least 3 h, alternatively at least 5 h.
**8.** The method of any one of embodiments 2-7, wherein the reaction is carried out in toluene at a temperature of 80-100°C and a pressure of 0.3-0.5 MPa for at least 5h, wherein the molar ratio of the compound of formula (II) and the ethylene oxide is 1:28.
**9.** A compound of formula (II): 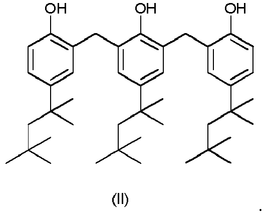
**10.** A method for preparing a compound of formula (II), 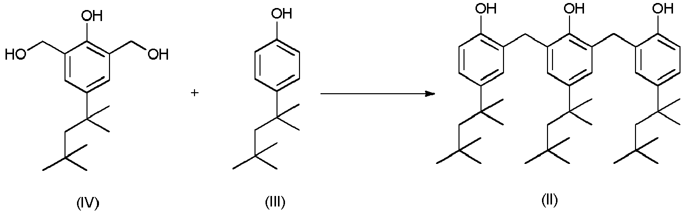 comprising the step of reacting a compound of formula (III) with a compound of formula (IV) under acidic conditions to obtain the compound of formula (II).
**11.** The method of embodiment 10, wherein the molar ratio of the compound of formula (IV) to the compound of formula (III) is less than 1:2, alternatively 1:2.1 to 1:3, yet alternatively 1:2.1 to 1:2.3, such as 1:2.1, 1:2.2 or 1:2.3.
**12.** The method of embodiment 10 or 11, wherein the reaction is carried out in oxalic acid, acetic acid, sulfuric acid or hydrochloric acid, alternatively in oxalic acid.
**13.** The method of any one of embodiments 10 to 12, wherein the reaction is carried out at 80°C or more, alternatively between 80 and 100°C, still alternatively at 100°C.
**14.** The method of any one of embodiments 10-13, wherein the reaction is carried out for at least 5h, alternatively 5-8h, alternatively at least 6h, still alternatively 6h.
**15.** The method of any one of embodiments 10-14, wherein the reaction conditions are one of the following:
   1) reacting under acidic conditions (such as in oxalic acid, acetic acid, sulfuric acid or hydrochloric acid) at a temperature of 80°C or more for at least 5 hours, wherein the molar ratio of the compound of formula (IV) to the compound of formula (III) is less than 1:2;
   2) reacting in glacial acetic acid at 100°C for at least 6 hours, wherein the molar ratio of the compound of formula (IV) to the compound of formula (III) is 1:2.2;
   3) reacting in 2 mol/L sulfuric acid at 100°C for at least 6 hours, wherein the molar ratio of the compound of formula (IV) to the compound of formula (III) is 1:2.2;
   4) reacting in 2 mol/L oxalic acid at 100°C for at least 6 hours, wherein the molar ratio of the compound of formula (IV) to the compound of formula (III) is 1:2.2;
   5) reacting in 32% hydrochloric acid at 100°C for at least 6 hours, wherein the molar ratio of the compound of formula (IV) to the compound of formula (III) is 1:2.2.
**16.** A method for preparing a compound of formula (IV), 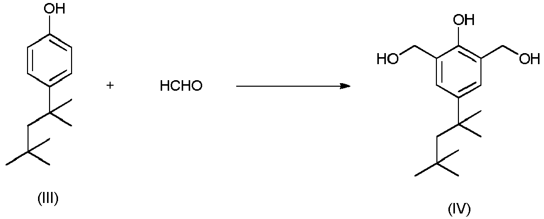 comprising the step of reacting the compound of formula (III) with formaldehyde under alkaline conditions to obtain the compound of formula (IV).
**17.** The method of embodiment 16, wherein the molar ratio of the compound of formula (III) to formaldehyde is less than 1:2, alternatively 1:2.1 to 1:5, yet alternatively 1:2.1 to 1:2.3, such as 1:2.1, 1:2.2 or 1:2.3.
**18.** The method of embodiment 16 or 17, wherein the molar ratio of the compound of formula (III) to a base is less than 1:1, alternatively 1:2 to 1:4, alternatively 1:2 to 1:3, yet alternatively 1:2.5.
**19.** The method of any one of embodiments 16 to 18, wherein the reaction is carried out in the presence of an inorganic base, alternatively an alkali metal hydroxide, such as lithium hydroxide, sodium hydroxide or potassium hydroxide.
**20.** The method of any one of embodiments 16 to 19, wherein the reaction is carried out at a temperature of 60°C or more, alternatively between 80 and 100°C.
**21.** The method of any one of embodiments 16 to 20, wherein the reaction is carried out for at least 2 h, alternatively at least 3 h, alternatively at least 4 h.
**22.** The method of any one of embodiments 16-20, wherein the reaction conditions are one of the following:
   1) in the presence of an inorganic base (alternatively an alkali metal hydroxide), reacting at 60°C or more for at least 3 hours, wherein the molar ratio of the compound of formula (III) to formaldehyde is less than 1:2, and the molar ratio of the compound of formula (III) to the inorganic base is less than 1:1;
   2) in the presence of potassium hydroxide, reacting at 80-100°C for at least 3 hours, wherein the molar ratio of the compound of formula (III) to formaldehyde is 1:2.2, and the molar ratio of the compound of formula (III) to potassium hydroxide is 1:2.5;
   3) in the presence of sodium hydroxide, reacting at 80-100°C for at least 3 hours, wherein the molar ratio of the compound of formula (III) to formaldehyde is 1:2.2, and the molar ratio of the compound of formula (III) to sodium hydroxide is 1:2.5.
**23.** A method for preparing a compound of formula (I), 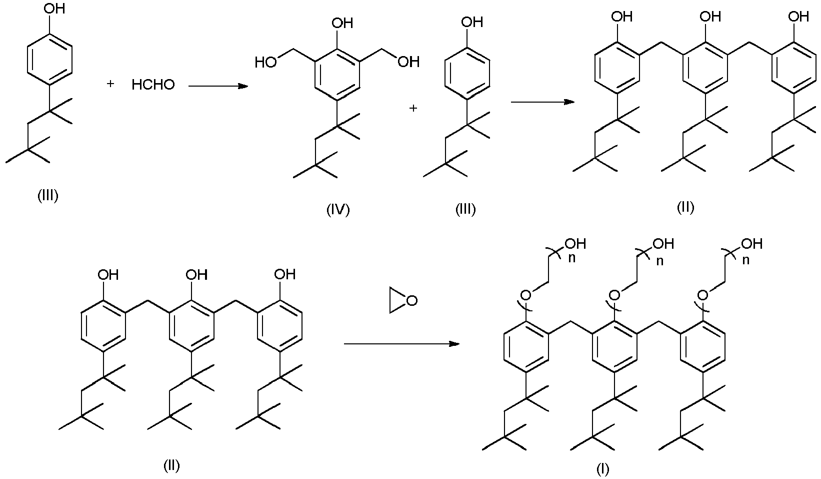 comprising the following steps:
   1) reacting the compound of formula (III) with formaldehyde at 80-100°C for at least 3 hours in the presence of sodium hydroxide or potassium hydroxide; wherein the molar ratio of the compound of formula (III) to formaldehyde is 1:2.2, and the molar ratio of the compound of formula (III) to sodium hydroxide/potassium hydroxide is 1:2.5;
   2) reacting the compound of formula (III) with the compound of formula (IV) in 2 mol/L oxalic acid at 100°C for at least 6 hours; wherein the molar ratio of the compound of formula (IV) to the compound of formula (III) is 1:2.2;
   3) reacting the compound of formula (II) with ethylene oxide in toluene at a temperature of 100°C and a pressure of 0.3-0.5 MPa for at least 5 hours, wherein the molar ratio of the compound of formula (II) to ethylene oxide is 1:28.

Beneficial effects of the present disclosure:
The new method of the present disclosure has the advantages of simple operation, mild reaction conditions, high purity of reaction products, high yield, etc., and is beneficial to industrial production.

### DETAILED DESCRIPTION

### Definition

"Aprotic solvent" is also called non-proton transfer solvent or non-protonic solvent. The autoprotolysis reaction of such solvents is extremely weak or has no autoprotolysis tendency. Aprotic solvents can be divided into: aprotic non-polar solvents, such as alkanes, such as n-hexane, heptane, benzene, toluene, diethyl ether, carbon tetrachloride, etc.; aprotic polar solvents, such as amides, ketones, nitriles, dimethyl sulfoxide, pyridine, dichloromethane, N,N-dimethylformamide, acetone, etc.

"Acidic conditions" refers to conditions in which the pH of a system is less than 7, usually achieved by adding acid. Acids include organic acids and inorganic acids. Organic acids include carboxylic acid (R-COOH), sulfonic acid (R-SO₃H), sulfinic acid (R-SOOH), thiocarboxylic acid (R-SH), etc., such as acetic acid, tartaric acid, oxalic acid, malic acid, citric acid, ascorbic acid, benzoic acid, salicylic acid, etc.; inorganic acids include hydrochloric acid, sulfuric acid, nitric acid, phosphoric acid, etc.

"Alkaline conditions" refers to conditions in which the pH of a system is greater than 7, usually achieved by adding a base. Bases include organic bases and inorganic bases, wherein organic bases include amine compounds and nitrogen-containing heterocyclic compounds, also include generalized organic bases, such as alkali metal salts of alcohols, such as sodium methoxide, potassium ethoxide, and potassium tert-butoxide, alkyl lithium compounds, such as butyl lithium and phenyl lithium, lithium amide compounds, such as lithium diisopropylamide, lithium hexamethyldisilazide, etc.; inorganic bases include lithium hydroxide, sodium hydroxide, potassium hydroxide, cesium hydroxide, barium hydroxide, calcium hydroxide, ammonium hydroxide, potassium carbonate, sodium carbonate, lithium carbonate, cesium carbonate, potassium bicarbonate, sodium bicarbonate, potassium bicarbonate, cesium bicarbonate, etc.

The technical solution of the present disclosure will be clearly and completely described below with reference to the examples. Obviously, the described examples are only used to illustrate the present disclosure, but not to limit the present disclosure. Based on the examples of the present disclosure, all other examples obtained by those of ordinary skill in the art without making creative efforts fall within the scope of protection of the present disclosure.

The method for synthesizing tyloxapol is as follows: 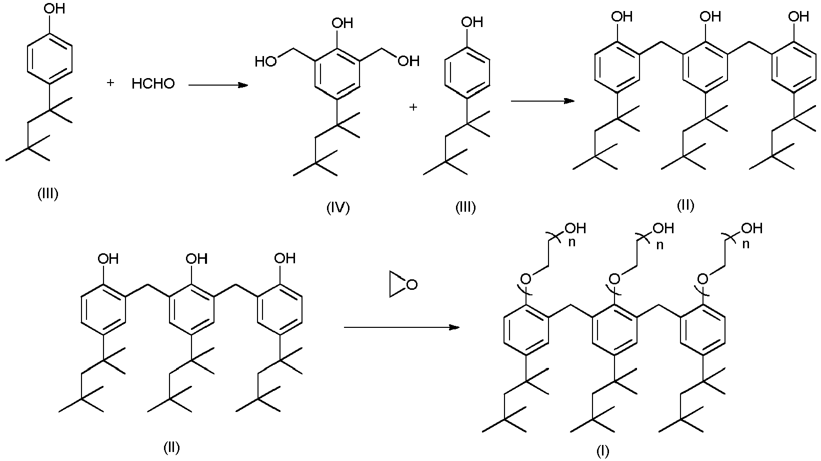 wherein n is 6, 7, 8, 9, 10, 11 or 12; alternatively, n is 8, 9 or 10; yet alternatively, n is 9.

Step 1: The compound of formula (III) is reacted with formaldehyde under alkaline conditions to obtain the compound of formula (IV);

Step 2: The compound of formula (IV) is reacted with the compound of formula (III) under acidic conditions to obtain the compound of formula (II);

Step 3: The compound of formula (II) is reacted with ethylene oxide to obtain the compound of formula (I).

### Example

### Example 1

### Synthesis of 2,5-dihydroxymethyl p-tert-octylphenol

103.0 g of p-tert-octylphenol and 33 g of formaldehyde were added into a reaction bottle. 500 mL of water was added into the reaction bottle, and 50.0 g of sodium hydroxide was added in batches into the reaction bottle. After the addition was completed, the temperature was raised to 100°C, and the reaction was stirred for 3 hours. After the reaction was completed, the reaction solution was extracted with dichloromethane to obtain an organic phase. The organic phase was washed once respectively with clean water and saturated brine, and evaporated to dryness to obtain a yellow liquid, 117.6 g, with a yield of 88.4%. ESI-MS[M + H]⁺ = 267.5.

### Test 1: Effects of different bases on the preparation of the compound of formula (IV).

**Table 1: Investigation of the effects of different bases on the preparation of the compound of formula (IV)**

| | Example 1 | Example 2 | Example 3 | Comparative example 1 |
|---|---|---|---|---|
| Inorganic base used | Sodium hydroxide | Lithium hydroxide | Potassium hydroxide | Potassium carbonate |
| Reaction temperature (°C) | 100 | 100 | 100 | 100 |
| The feeding ratio of formaldehyde (the molar ratio of the compound of formula (III) to formaldehyde) | 1:2.2 | 1:2.2 | 1:2.2 | 1:2.2 |
| The feeding ratio of base (the molar ratio of the compound of formula (III) to base) | 1:2.5 | 1:2.5 | 1:2.5 | 1:2.5 |

**Table 2: Results of the effects of different bases on the preparation of the compound of formula (IV)**

| | Example 1 | Example 2 | Example 3 | Comparative example 1 |
|---|---|---|---|---|
| Product mass (g) | 117.6 | 88.2 | 111.3 | No reaction |
| Yield (%) | 88.4 | 66.3 | 83.5 | |
| ESI-MS[M+H]+ | 267.2 | 267.5 | 267.2 | |

When the alkalinity of the base used is weak, the reaction cannot proceed normally. Therefore, alkali metal hydroxides can be used, alternatively sodium hydroxide or potassium hydroxide.

Test 2: Effect of different reaction temperatures on the preparation of the compound of formula (IV).

**Table 3: Investigation of the effects of different reaction temperatures on the preparation of the compound of formula (IV)**

| | Example 1 | Example 4 | Example 5 | Comparative example 2 |
|---|---|---|---|---|
| Inorganic base used | Sodium hydroxide | Sodium hydroxide | Sodium hydroxide | Sodium hydroxide |
| Reaction temperature (°C) | 100 | 90 | 80 | 60 |
| The feeding ratio of formaldehyde (the molar ratio of the compound of formula (III) to formaldehyde) | 1:2.2 | 1:2.2 | 1:2.2 | 1:2.2 |
| The feeding ratio of base (the molar ratio of the compound of formula (III) to base) | 1:2.5 | 1:2.5 | 1:2.5 | 1:2.5 |

**Table 4: Results of the effects of different reaction temperatures on the preparation of the compound of formula (IV)**

| | Example 1 | Example 4 | Example 5 | Comparative example 2 |
|---|---|---|---|---|
| Product mass (g) | 117.6 | 109.5 | 112.1 | 43.4 |
| Yield (%) | 88.4 | 82.3 | 84.2 | 32.6 |
| ESI-MS[M+H]+ | 267.2 | 267.2 | 267.2 | 267.2 |

The above test results prove that this reaction requires a higher temperature to proceed, and the reaction cannot proceed completely within the same time when the temperature is too low. Therefore, the selected temperature should be 60°C or more, alternatively 80-100°C.

Test 3: Effect of the feeding ratio of formaldehyde on the preparation of the compound of formula (IV).

**Table 5: Investigation of the effects of the feeding ratio of formaldehyde on the preparation of the compound of formula (IV)**

| | Example 6 | Example 7 | Example 1 | Example 8 |
|---|---|---|---|---|
| Inorganic base used | Sodium hydroxide | Sodium hydroxide | Sodium hydroxide | Sodium hydroxide |
| Reaction temperature (°C) | 100 | 100 | 100 | 100 |
| The feeding ratio of formaldehyde (the molar ratio of the compound of formula (III) to formaldehyde) | 1:2 | 1:2.1 | 1:2.2 | 1:5 |
| The feeding ratio of base (the molar ratio of the compound of formula (III) to base) | 1:2.5 | 1:2.5 | 1:2.5 | 1:2.5 |

**Table 6: Results of the effects of the feeding ratio of formaldehyde on the preparation of the compound of formula (IV)**

| | Example 6 | Example 7 | Example 1 | Example 8 |
|---|---|---|---|---|
| Product mass (g) | 86.7 | 111.0 | 117.6 | 118.1 |
| Yield (%) | 65.2 | 83.4 | 88.4 | 88.8 |
| ESI-MS[M+H]+ | 267.2 | 267.2 | 267.2 | 267.2 |

The above test results prove that the amount of formaldehyde required for this reaction needs to be slightly greater than 2 times of a standard amount to ensure the stability of the yield. However, further increase of the amount of the formaldehyde cannot significantly improve the yield. Therefore, the selected amount of formaldehyde should be >2 times, alternatively 2.1-5 times, yet alternatively 2.1-2.3 times.

Test 4: Effect of the feeding ratio of base on the preparation of the compound of formula (IV).

**Table 7: Investigation of the effects of the feeding ratio of base on the preparation of the compound of formula (IV)**

| | Example 9 | Example 1 | Example 10 | Example 11 |
|---|---|---|---|---|
| Inorganic base used | Sodium hydroxide | Sodium hydroxide | Sodium hydroxide | Sodium hydroxide |
| Reaction temperature (°C) | 100 | 100 | 100 | 100 |
| The feeding ratio of formaldehyde (the molar ratio of the compound of formula (III) to formaldehyde) | 1:2.2 | 1:2.2 | 1:2.2 | 1:2.2 |
| The feeding ratio of base (the molar ratio of the compound of formula (III) to base) | 1:2 | 1:2.5 | 1:3 | 1:4 |

**Table 8: Results of the effects of the feeding ratio of base on the preparation of the compound of formula (IV)**

| | Example 9 | Example 1 | Example 10 | Example 11 |
|---|---|---|---|---|
| Product mass (g) | 107.6 | 117.6 | 113.1 | 110.9 |
| Yield (%) | 80.9 | 88.4 | 85.0 | 83.4 |
| ESI-MS[M+H]+ | 267.2 | 267.2 | 267.2 | 267.2 |

The above test results show that different amounts of bases have little effect on the yield of the product. In order to ensure the completeness of the reaction, it is determined that the molar ratio of the compound of formula (III) to the base should be less than 1:1, alternatively 1:2 to 1:4. alternatively 1:2 to 1:3, yet alternatively 1:2.5.

### Example 12

### Synthesis of phenolic resin

66.5 g of 2,5-dihydroxymethyl p-tert-octylphenol and 113.3 g of p-tert-octylphenol were added into a reaction bottle. 250 mL of purified water and 54g of oxalic acid were added into the mixture. After the addition was completed, the temperature was raised to 100°C and the reaction was stirred for 6 h. After the reaction was completed, the pH was adjusted to 7-8 by 1mol/L sodium hydroxide aqueous solution. The aqueous solution was extracted with dichloromethane to obtain an organic phase. The organic phase was washed once respectively with clean water and saturated brine, and the organic phase was evaporated to dryness to obtain a dark yellow liquid, 127.7 g, yield 79.6%. ¹H NMR (400 MHz, CDCl₃₎ δ 7.77 (s, 2H), 7.25 (d, *J* = 2.5 Hz, 3H), 7.10-6.59 (m, 5H), 3.89 (d, *J* = 12.5 Hz, 3H), 1.67 (d, *J* = 5.4 Hz, 6H), 1.31 (d, *J* = 5.6 Hz, 18H), 0.68 (d, *J* = 5.9 Hz, 27H).

### Test 5: Effects of a feeding ratio on the preparation of the compound of formula (II)

**Table 9: Investigation of the effects of a feeding ratio on the preparation of the compound of formula (II)**

| | Example 13 | Example 12 | Example 14 | Example 15 |
|---|---|---|---|---|
| The acid used | 2mol/L oxalic acid | 2mol/L oxalic acid | 2mol/L oxalic acid | 2mol/L oxalic acid |
| Reaction temperature (°C) | 100 | 100 | 100 | 100 |
| Feed ratio (the molar ratio of the compound of formula (IV) to the compound of formula (III)) | 1:2 | 1:2.2 | 1:2.4 | 1:3 |
| Reaction time (h) | 6 | 6 | 6 | 6 |

**Table 10: Results of the effects of the feeding ratio on the preparation of the compound of formula (II)**

| | Example 13 | Example 12 | Example 14 | Example 15 |
|---|---|---|---|---|
| Product mass (g) | 117.4 | 127.7 | 123.2 | 126.6 |
| Yield (%) | 73.2 | 79.6 | 76.8 | 78.9 |

The above test results show that when the feed ratio is 1:2, there will be residual raw materials. The feed ratio required for this reaction needs to be slightly greater than 2 times of a standard amount to ensure the stability of the yield. However, further increase of the feed ratio cannot greatly improve the yield. Therefore, it is appropriate to select a feed ratio of >2 times, alternatively 2.1-3 times, yet alternatively 2.1-2.3 times, such as 2.1, 2.2 or 2.3 times.

Test 6: Effects of different acids on the preparation of the compound of formula (II)

**Table 11: Investigation of the effects of different acids on the preparation of the compound of formula (II)**

| | Example 12 | Example 16 | Example 17 | Example 18 |
|---|---|---|---|---|
| The acid used | 2mol/L oxalic acid | 2mol/L sulfuric acid | glacial acetic acid | 32% hydrochloric acid |
| Reaction temperature (°C) | 100 | 100 | 100 | 100 |
| Feed ratio (the molar ratio of the compound of formula (IV) to the compound of formula (III)) | 1:2.2 | 1:2.2 | 1:2.2 | 1:2.2 |
| Reaction time (h) | 6 | 6 | 6 | 6 |

**Table 12: Results of the effects of different acid ratios on the preparation of the compound of formula (II)**

| | Example 12 | Example 16 | Example 17 | Example 18 |
|---|---|---|---|---|
| Product mass (g) | 127.7 | 117.1 | 100.2 | 98.7 |
| Yield (%) | 79.6 | 73.0 | 62.5 | 61.6 |

The above tests prove that the reaction can proceed smoothly under acidic conditions, and the reaction result of oxalic acid is the best. Therefore, oxalic acid is preferred.

### Test 7: Effects of reaction time on the preparation of the compound of formula (II)

**Table 13: Investigation of the effects of reaction time on the preparation of the compound of formula (II)**

| | Example 19 | Example 12 | Example 20 | Example 21 |
|---|---|---|---|---|
| The acid used | 2mol/L oxalic acid | 2mol/L oxalic acid | 2mol/L oxalic acid | 2mol/L oxalic acid |
| Reaction temperature (°C) | 100 | 100 | 100 | 100 |
| Feed ratio (the molar ratio of the compound of formula (IV) to the compound of formula (III)) | 1:2.2 | 1:2.2 | 1:2.2 | 1:2.2 |
| Reaction time (h) | 5 | 6 | 7 | 8 |

**Table 14: Results of the effects of reaction time on the preparation of the compound of formula (II)**

| | Example 19 | Example 12 | Example 20 | Comparative example 21 |
|---|---|---|---|---|
| Product mass (g) | 124.3 | 127.7 | 124.0 | 130.2 |
| Yield (%) | 77.5 | 79.6 | 77.3 | 81.2 |

The above tests prove that when the reaction time is not less than 5h, the reaction can proceed smoothly. After extending the reaction time, the yield does not change much. Therefore, in order to ensure the complete progress of the reaction, it is confirmed that the reaction time should be at least 5h, alternatively 5-8h, alternatively at least 6h, yet alternatively 6h.

### Example 22

### Synthetic method of tyloxapol

80.0 g of phenolic resin and 400.0 mL of toluene were added into a pressure-resistant reaction bottle. The reaction was cooled to 0°C. 153.2g of ethylene oxide was added to the reaction, and the reaction was heated to 80-100°C in a sealed reaction bottle. At this time, the pressure in the reaction bottle was about 0.3-0.5 MPa. The reaction was carried out for 5 h while maintaining this temperature. After the reaction was completed, the reaction solution was cooled to 0°C. 400 mL of water was added to the reaction solution, and the pH was adjusted to 9 with 1 mol/L sodium hydroxide aqueous solution. The liquid was separated to obtain an organic phase. The organic phase was washed once with water and saturated brine respectively. The organic phase was collected and the solvent was evaporated to dryness to afford a yellow oil. The obtained yellow oil was distilled under reduced pressure to afford 76.0 g of a light-yellow oil, with a yield of 77.3% and a purity of 99.4%. ¹H NMR (400 MHz, DMSO-*d₆₎* δ7.54-6.41 (m, 8H), 4.54 (t, *J* = 5.4 Hz, 3H), 4.28-3.78 (m, 4H), 3.69 (ddt, *J* = 14.5, 9.3, 4.1 Hz, 10H), 3.76-3.40 (m, 98H), 1.92-1.45 (m, 6H), 1.36-1.04 (m, 18H), 0.88-0.27 (m, 27H).

### Test 8: Exploration of the molar ratio of the compound of formula (II) to ethylene oxide

**Table 15: Test examples of exploring the molar ratio of the compound of formula (II) to ethylene oxide**

| | Example 23 | Example 22 | Example 24 | Example 25 | Example 26 |
|---|---|---|---|---|---|
| The molar ratio of the compound of formula (II) to ethylene oxide | 1:31 | 1:28 | 1:25 | 1:22 | 1:19 |
| Reaction temperature (°C) | 100 | 100 | 100 | 100 | 100 |
| Reaction pressure (MPa) | 0.3 | 0.3 | 0.3 | 0.3 | 0.3 |
| Reaction time (h) | 5 | 5 | 5 | 5 | 5 |

T

**able 16: Results of exploring the molar ratio of the compound of formula (II) to ethylene oxide**

| | Example 23 | Example 22 | Example 24 | Example 25 | Example 26 |
|---|---|---|---|---|---|
| Product name (n value) and appearance | n=10 | n=9 | n=8 | n=7 | n=6 |
| Product mass (g) | 82.1 | 76.0 | 66.4 | 59.9 | 56.8 |
| Yield (%) | 77.9 | 77.3 | 78.1 | 76.4 | 79.2 |

The above test results prove that the degree of polymerization of the compound can be controlled by the molar ratio of the compound of formula (II) to ethylene oxide. In order to obtain a product with a degree of polymerization of 9, an alternative feeding ratio is 1:27 to 1:29, alternatively 1:28.

### Test 9: Exploration of reaction temperature

**Table 17: Test examples of exploring the reaction temperature**

| | Example 27 | Example 28 | Example 22 | Example 29 |
|---|---|---|---|---|
| The molar ratio of the compound of formula (II) to ethylene oxide | 1:28 | 1:28 | 1:28 | 1:28 |
| Reaction temperature (°C) | 80 | 90 | 100 | 110 |
| Reaction pressure (MPa) | 0.3 | 0.3 | 0.3 | 0.3 |
| Reaction time (h) | 5 | 5 | 5 | 5 |

**Table 18: Results of exploring the reaction temperature**

| | Example 27 | Example 28 | Example 22 | Example 29 |
|---|---|---|---|---|
| Product mass (g) | 71.9 | 71.3 | 76.0 | 75.8 |
| Yield (%) | 73.1 | 72.5 | 77.3 | 77.1 |

The above test results show that all the reactions can proceed normally. Therefore, it is confirmed that the temperature may be higher than 60°C (such as 60-120°C), alternatively higher than 80°C (such as 80-100°C), or yet alternatively 100°C.

### Test 10: Exploration of reaction pressure

**Table 19: Test example of exploring the reaction pressure**

| | Comparative example 3 | Example 22 | Example 30 | Example 31 |
|---|---|---|---|---|
| The molar ratio of the compound of formula (II) to ethylene oxide | 1:28 | 1:28 | 1:28 | 1:28 |
| Reaction temperature (°C) | 100 | 100 | 100 | 110 |
| Reaction pressure (MPa) | 0.1 | 0.3 | 0.5 | 0.8 |
| Reaction time (h) | 5 | 5 | 5 | 5 |

**Table 20: Results of exploring the reaction pressure**

| | Comparative example 3 | Example 22 | Example 30 | Example 31 |
|---|---|---|---|---|
| Product mass (g) | Incorrect product | 76.0 | 77.8 | 76.7 |
| Yield (%) | | 77.3 | 79.1 | 78.0 |

The above results prove that the reaction needs to be carried out under pressure. Under normal pressure, only one ethylene oxide is reacted, but polymerization cannot occur. Therefore, the reaction must be pressurized. However, the higher the pressure, the greater the reaction risk. Through investigation, it is finally confirmed that the pressure may be 0.2-0.8 MPa, alternatively 0.3-0.8MPa, yet alternatively 0.3-0.5MPa.

Although the present disclosure has been disclosed above in terms of preferred and alternative examples, they are not intended to limit the present disclosure. Anyone familiar with this technology can make various changes and modifications without departing from the spirit and scope of the present disclosure. Therefore, the protection scope of the present disclosure should be defined by the claims.

## Claims

1. A compound of formula (I): 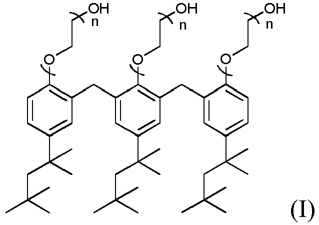 wherein n is 6, 7, 8, 9, 10, 11 or 12; alternatively, n is 8, 9 or 10; yet alternatively, n is 9.

2. A method for preparing the compound of formula (I) of claim 1, 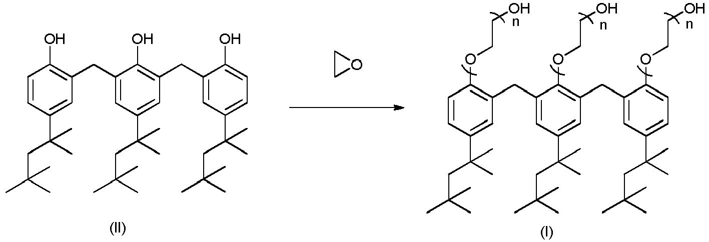 comprising the step of reacting the compound of formula (II) with ethylene oxide to obtain the compound of formula (I).

3. The method of claim 2, wherein the molar ratio of the compound of formula (II) to ethylene oxide is 1:19 to 1:37, alternatively 1:25 to 1:32, alternatively 1:27 to 1:29, yet alternatively 1:28.

4. The method of claim 2 or 3, wherein the reaction of the compound of formula (II) with ethylene oxide is carried out in an aprotic solvent, alternatively in an aprotic non-polar solvent; alternatively, the solvent is an alkane, such as n-hexane, heptane, benzene, and toluene, still alternatively, toluene.

5. The method of any one of claims 2-4, wherein the reaction is carried out at a temperature of 60°C or more (such as 60-120°C), alternatively at a temperature of 80°C or more (such as 80-100°C), still alternatively at a temperature of 100°C.

6. The method of any one of claims 2-5, wherein the reaction is carried out at a pressure of 0.2-0.8 MPa, alternatively at a pressure of 0.3-0.8 MPa, yet alternatively at a pressure of 0.3-0.5 MPa.

7. The method of any one of claims 2-6, wherein the reaction is carried out for at least 3 h, alternatively at least 5 h.

8. The method of any one of claims 2-7, wherein the reaction is carried out in toluene at a temperature of 80-100°C and a pressure of 0.3-0.5 MPa for at least 5h, wherein the molar ratio of the compound of formula (II) and the ethylene oxide is 1:28.

9. A compound of formula (II):

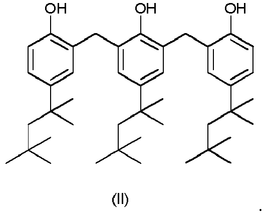

10. A method for preparing a compound of formula (II), 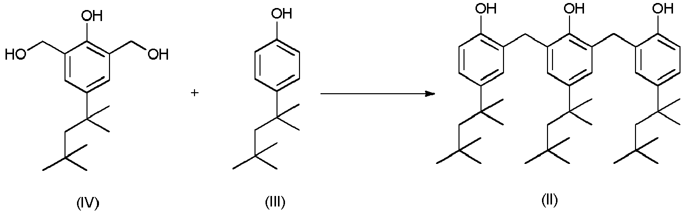 comprising the step of reacting a compound of formula (III) with a compound of formula (IV) under acidic conditions to obtain the compound of formula (II).

11. The method of claim 10, wherein the molar ratio of the compound of formula (IV) to the compound of formula (III) is less than 1:2, alternatively 1:2.1 to 1:3, yet alternatively 1:2.1 to 1:2.3, such as 1:2.1, 1:2.2 or 1:2.3.

12. The method of claim 10 or 11, wherein the reaction is carried out in oxalic acid, acetic acid, sulfuric acid or hydrochloric acid, alternatively in oxalic acid.

13. The method of any one of claims 10 to 12, wherein the reaction is carried out at 80°C or more, alternatively between 80 and 100°C, still alternatively at 100°C.

14. The method of any one of claims 10-13, wherein the reaction is carried out for at least 5h, alternatively 5-8h, alternatively at least 6h, still alternatively 6h.

15. The method of any one of claims 10-14, wherein the reaction conditions are one of the following:
1) reacting under acidic conditions (such as in oxalic acid, acetic acid, sulfuric acid or hydrochloric acid) at a temperature of 80°C or more for at least 5 hours, wherein the molar ratio of the compound of formula (IV) to the compound of formula (III) is less than 1:2;
2) reacting in glacial acetic acid at 100°C for at least 6 hours, wherein the molar ratio of the compound of formula (IV) to the compound of formula (III) is 1:2.2;
3) reacting in 2 mol/L sulfuric acid at 100°C for at least 6 hours, wherein the molar ratio of the compound of formula (IV) to the compound of formula (III) is 1:2.2;
4) reacting in 2 mol/L oxalic acid at 100°C for at least 6 hours, wherein the molar ratio of the compound of formula (IV) to the compound of formula (III) is 1:2.2;
5) reacting in 32% hydrochloric acid at 100°C for at least 6 hours, wherein the molar ratio of the compound of formula (IV) to the compound of formula (III) is 1:2.2.

16. A method for preparing a compound of formula (IV), 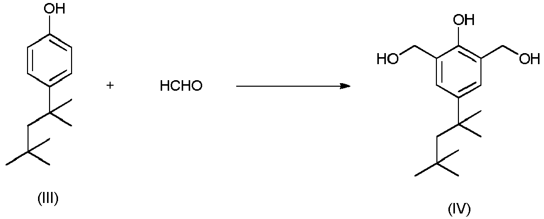 comprising the step of reacting the compound of formula (III) with formaldehyde under alkaline conditions to obtain the compound of formula (IV).

17. The method of claim 16, wherein the molar ratio of the compound of formula (III) to formaldehyde is less than 1:2, alternatively 1:2.1 to 1:5, yet alternatively 1:2.1 to 1:2.3, such as 1:2.1, 1:2.2 or 1:2.3.

18. The method of claim 16 or 17, wherein the molar ratio of the compound of formula (III) to a base is less than 1:1, alternatively 1:2 to 1:4, alternatively 1:2 to 1:3, yet alternatively 1:2.5.

19. The method of any one of claims 16 to 18, wherein the reaction is carried out in the presence of an inorganic base, alternatively an alkali metal hydroxide, such as lithium hydroxide, sodium hydroxide or potassium hydroxide.

20. The method of any one of claims 16 to 19, wherein the reaction is carried out at a temperature of 60°C or more, alternatively between 80 and 100°C.

21. The method of any one of claims 16 to 20, wherein the reaction is carried out for at least 2 h, alternatively at least 3 h, alternatively at least 4 h.

22. The method of any one of claims 16-20, wherein the reaction conditions are one of the following:
1) in the presence of an inorganic base (alternatively an alkali metal hydroxide), reacting at 60°C or more for at least 3 hours, wherein the molar ratio of the compound of formula (III) to formaldehyde is less than 1:2, and the molar ratio of the compound of formula (III) to the inorganic base is less than 1: 1;
2) in the presence of potassium hydroxide, reacting at 80-100°C for at least 3 hours, wherein the molar ratio of the compound of formula (III) to formaldehyde is 1:2.2, and the molar ratio of the compound of formula (III) to potassium hydroxide is 1:2.5;
3) in the presence of sodium hydroxide, reacting at 80-100°C for at least 3 hours, wherein the molar ratio of the compound of formula (III) to formaldehyde is 1 :2.2, and the molar ratio of the compound of formula (III) to sodium hydroxide is 1:2.5.

23. A method for preparing a compound of formula (I), 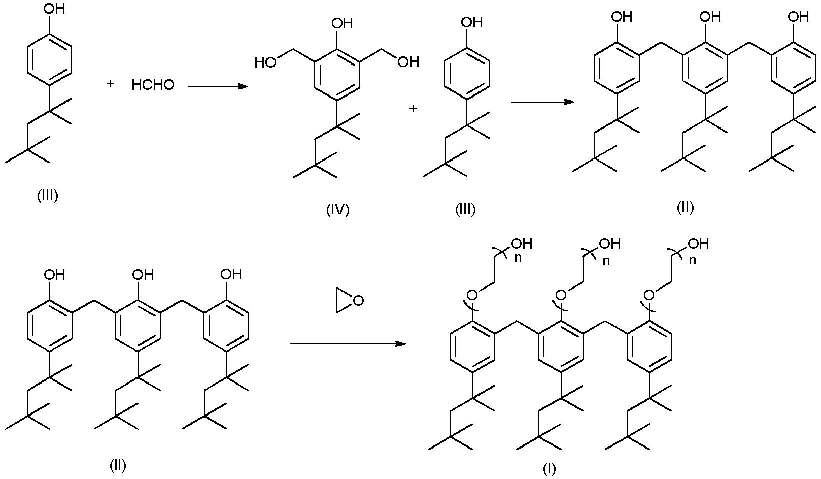 comprising the following steps:
1) reacting the compound of formula (III) with formaldehyde at 80-100°C for at least 3 hours in the presence of sodium hydroxide or potassium hydroxide; wherein the molar ratio of the compound of formula (III) to formaldehyde is 1:2.2, and the molar ratio of the compound of formula (III) to sodium hydroxide/potassium hydroxide is 1:2.5;
2) reacting the compound of formula (III) with the compound of formula (IV) in 2 mol/L oxalic acid at 100°C for at least 6 hours; wherein the molar ratio of the compound of formula (IV) to the compound of formula (III) is 1:2.2;
3) reacting the compound of formula (II) with ethylene oxide in toluene at a temperature of 100°C and a pressure of 0.3-0.5 MPa for at least 5 hours, wherein the molar ratio of the compound of formula (II) to ethylene oxide is 1:28.
